# FASCICULE DE BREVET EUROPEEN

(11) **EP 1 337 261 B1**
(45) Date de publication et mention de la délivrance du brevet: **21.03.2007**
(21) Numéro de dépôt: 01992577.5
(22) Date de dépôt: 02.11.2001
(51) Int. Cl.: A61K 31/737, A61P 29/00

(54) **MEDICAMENT ANTI-INFLAMMATOIRE A BASE DE SULFATE DE LAMINARINE**
ENTZÜNDUNGSHEMMENDES MEDIKAMENT AUF LAMINARINSULFAT-BASIS
LAMINARIN SULPHATE-BASED ANTI-INFLAMMATORY MEDICAMENT

(30) Priorité: 03.11.2000 FR 0014118
(43) Date de publication de la demande: 27.08.2003
(73) Titulaire: LABORATOIRES GOEMAR S.A., 35400 Saint-Malo (FR)
(72) Inventeur: YVIN, Jean-Claude, F-35400 Saint-Malo (FR); ALBAN, Susanne, 24146 Kiel (DE); FRANZ, Gerhard, 93049 Regensburg (DE)
(74) Mandataire: Koch, Gustave
(86) Numéro de dépôt international: PCT/FR2001/003397
(87) Numéro de publication internationale: WO 2002/036132

(56) Documents cités:
- WO-A-95/24907
- US-A- 5 135 920
- PAPER D H (REPRINT): "Natural products as angiogenesis inhibitors" PLANTA MEDICA, (DEC 1998) VOL. 64, NO. 8, PP. 686-695. PUBLISHER: GEORG THIEME VERLAG, P O BOX 30 11 20, D-70451 STUTTGART, GERMANY. ISSN: 0032-0943., XP002176352 UNIV REGENSBURG, DEPT PHARM, UNIV STR 31, D-93040 REGENSBURG, GERMANY (Reprint)
- HOFFMANN R ET AL: "Inhibition of angiogenesis and murine tumour growth by laminarin sulphate." BRITISH JOURNAL OF CANCER, vol. 73, no. 10, 1996, pages 1183-1186, XP001053335 ISSN: 0007-0920 cité dans la demande
- YOSHIDA, TOMOAKI ET AL: "A liquid - phase binding analysis for L- selectin--. A strong dependency of highly clustered sulfate groups" EUR. J. BIOCHEM. (1994), 222(2), 703-9, XP001016234
- XIE X ET AL: "INHIBITION OF SELECTIN-MEDIATED CELL ADHESION AND PREVENTION OF ACUTE INFLAMMATION BY NONANTICOAGULANT SULFATED SACCHARIDES STUDIES WITH CARBOXYL-REDUCED AND SULFATED HEPARIN AND WITH TRESTATIN A SULFATE" JOURNAL OF BIOLOGICAL CHEMISTRY, AMERICAN SOCIETY OF BIOLOGICAL CHEMISTS, BALTIMORE, MD, US, vol. 275, no. 44, 3 novembre 2000 (2000-11-03), pages 34818-34825, XP001031395 ISSN: 0021-9258
- SUZUKI, TATSUYA ET AL: "Preparation and biological activities of sulfated derivatives of (1.fwdarw. 3)-.beta.-D- glucans" J. PHARMACOBIO -DYN. (1991), 14(5), 256-66, XP001016209
- DATABASE CA [en ligne] CHEMICAL ABSTRACTS SERVICE, COLUMBUS, OHIO, US; HAMURO, JUNJI ET AL: "Polysaccharide sulfuric acid esters" retrieved from STN Database accession no. 83:12692 XP002176355 & JP 49 130478 A (AJINOMOTO CO., INC.) 13 décembre 1974 (1974-12-13)
- DATABASE CA [en ligne] CHEMICAL ABSTRACTS SERVICE, COLUMBUS, OHIO, US; MINAMI, S. ET AL: "Veterinary-clinical uses of curdlan sulfate" retrieved from STN Database accession no. 132:73593 XP002176356 & KICHIN, KITOSAN KENKYU (1999), 5(2), 166-167,
- DATABASE PHIN [en ligne] Agrow (2000) Newsletter No. 352 p 22, 19 mai 2000 (2000-05-19) "Goemar expects French approval for GL 32" XP002176357

## Description

Les problèmes liés au traitement des maladies inflammatoires correspondant à des réactions inflammatoires non spécifiques sont loin d'être résolus.

En effet, les médicaments proposés à cet effet présentent tous des effets secondaires qui en limitent l'application.

Parmi ces médicaments, le praticien a souvent recours aux corticostéroïdes ou aux anti-inflammatoires non stéroïdiens (AINS) dont l'efficacité est souvent spectaculaire mais dont les inconvénients et la mauvaise compliance limitent l'utilisation.

Ces inconvénients se traduisent fréquemment pour les corticostéroïdes utilisés par voie générale par des troubles métaboliques, endocriniens, digestifs ainsi que par le réveil des infections. Par voie locale les corticoïdes peuvent induire des dermites, un effet rebond à l'arrêt du traitement ainsi qu'un développement favorisé des infections. Pour les AINS, on peut observer lorsqu'ils sont utilisés par voie locale, des réactions cutanées érythémateuses, des réactions allergiques locales de type eczéma et lorsqu'ils sont utilisés par voie générale, des troubles digestifs, des prurits, des éruptions cutanées.

Il existe donc un besoin permanent d'enrichir la panoplie actuelle de ces médicaments, si possible par des produits au moins aussi efficaces que les corticostéroïdes mais dépourvus autant que faire se peut des inconvénients de ces derniers.

C'est ce but qu'a poursuivi la Société Demanderesse.

Et elle a eu le mérite de trouver que, de façon surprenante et inattendue, un certain polysaccharide sulfaté répondait à ce besoin, à savoir le sulfate de laminarine qui est défini ci-après et qui est déjà connu pour d'autres activités pharmacologiques décrites dans les articles suivants :
- *"Inhibition of heparanase activity and tumor metastasis by laminarin sulfate and synthetic phosphorothioate oligodeoxynucleotides,* publié par Miao-HQ ; Elkin-M ; Aingorn-E, Ishai-Michaeli-R ; Stain-CA ; Vlodavsky-I dans Int-J-Cancer. 1999 Oct 29 ; 83(3) : 424-31, selon lequel les résultats obtenus soulignent l'implication de l'héparanase dans des métastases ainsi que les applications cliniques potentielles de diverses molécules inhinbitrices de l'héparanase telles que des polysaccharides sulfatés ;
- *"Inhibition of angiogenesis and murine tumour growth by laminarin sulphate"* publié par Hoffinan-R ; Paper-DH ; Donaldson-J ; Vogl-H dans Br-J-Cancer. 1996 May ; 73(10) : 1183-6, suivant lequel un dérivé polysulfaté de glucan ralentit la croissance de la tumeur murine RIF-1 de 2 à 6 jours ;
- *"Differential effects of polysulfated polysaccharide on experimental encephalomyelitis, proliferation of autoimmune T cells, and inhibition of heparanase activity"*, publié par Hershkoviz-R ; Mor-F ; Miao-HQ ; Vlodavsky-I ; Lider-O dans J-Autoimmun. 1995 Oct; 8(5): 741-50 et selon lequel des polysaccharides polysulfatés peuvent avoir des applications cliniques potentielles dans le traitement des maladies auto-immunes ;
- *"Characterisation of a laminarin sulphate which inhibits basic fibroblast growth factor binding and endothelial cell proliferation",* publié par Hoffman-R; Paper-DH ; Donaldson-J ; Alban-S; Franz-G dans J-Cell-Sci. 1995 Nov ; 108 (Pt 11): 3591-8 et selon lequel la laminarine fortement sulfatée peut être intéressante contre des maladies associées à une prolifération cellulaire bFGF dépendante ;
- *"Laminarin sulfate mimics the effects of heparin on smooth muscle cell proliferation and basic fibroblast growth factor-receptor binding and mitogenic activity"* publié par Miao-HQ; Ishai-Michaeli-R; Peretz-T ; Vlodavsky-I dans J-Cell-Physiol. 1995 Sep ; 164(3) : 482-90 et selon lequel le sulfate de laminarine peut avoir des applications cliniques potentielles dans diverses situations telles que la guérison des plaies, l'angiogénèse et l'athérosclérose ;

### « Synthesis of laminarin sulfates with anticoagulant activity », publié par Alban S, Kraus J, et franz G. dans Arzneimittel-Forsch/Drug res (1992) 42 ; 1005-1008;

- *"Effects of laminarin sulphate on experimental atherosclerosis and on serum lipids in rabbits during long-term intermittent cholesterol feeding"* publié par Besterman-EM dans Atherosclerosis. 1970 Jul-Aug ; 12(1); 85-96. ;
- *"Natural product as Angiogenesis Inhibitors",* publié par Dietrich H. Paper dans Planta Medica (Déc. 1998), vol. 64, n°8, pp.686-695, selon lequel un β-(1,3)glucan hautement sulfaté, obtenu par sulfatation de la laminarine, inhibe l'angiogénèse et présente une activité anti-tumorale à l'encontre de la tumeur RIF-1.

Par ailleurs, le document Yoshida Tomohaki et al., Eur. J. Biochem. 1994, 222(2), 703-9, décrit l'effet inhibiteur d'un certain nombre d'hydrates de carbone, dont le sulfate de laminarine d'un degré de substitution de 2,0 ou 1,6, sur la liaison spécifique entre la sélectine L et le fucoidan marqué radioactivement par une tyrosine iodée, étant souligné que les résultats obtenus précisément avec les sulfates de laminarine sont les plus faibles de tous les résultats obtenus, cet article n'incitant donc nullement à avoir recours au sulfate de laminarine pour le traitement de maladies inflammatoires.

Le document Xie X et al., Journal of Biological Chemistry, 2000, vol. 275, n° 44, pages 34818-34825 décrit l'effet inhibiteur de l'adhésion due aux sélectines par, d'une part, le sulfate de trestatine-A et d'autre part, des dérivés d'héparine ; or le sulfate de trestatine n'est pas un oligosaccharide mais un dérivé de sucre à faible poids moléculaire et ne peut donc être comparé du point de vue clinique au sulfate de laminarine ; de même, les dérivés d'héparine ne sont pas des oligosaccharides mais des mélanges variables hautement complexes de polysaccharides , de plus, ils sont d'origine animale et non végétale.

Enfin, le brevet japonais JP 49130478-A référencé dans DATABASE CA, Chemical Abstract Service, Colombus, Ohio, US, Hamuro, Junji et al. repris de STN Database 83:12692, XP0021176355, décrit l'activité de β-1,3-glycans sulfatés contre la coagulation sanguine et les inflammations, s'agissant là de polysaccharides ramifiés de poids moléculaire élevé, dont la structure moléculaire est fondamentalement différente de celle des composés visés par la présente invention. En plus des effets anticoagulants et antilipémiques, ce document décrit uniquement un effet physicochimique *in vitro* supplémentaire, à savoir l'action inhibitrice des substances en question sur la thermocoagulation de l'albumine de sérum de boeuf.

La laminarine est commercialisée par la Société Demanderesse sous la marque de fabrique « PHYCARINE ».

L'invention a donc pour objet l'utilisation pour la préparation d'un médicament pour le traitement des maladies inflammatoires relevant de réactions inflammatoires non spécifiques antigène-indépendantes, d'un polysaccharide sulfate particulier, à savoir le sulfate de laminarine, dont le degré de sulfatation est supérieur ou égal à 1,9 et de préférence de 2 à 2,5 et dont le degré de polymérisation, identique à celui de la molécule naturelle, est de 20 à 30 de préférence de 23 à 25.

Elle vise d'un point de vue plus général un médicament pour le traitement des maladies inflammatoires relevant de réactions inflammatoires non spécifiques antigène-indépendantes, ce médicament comportant en tant que substance active une concentration efficace d'un polysaccharide sulfate particulier, à savoir le sulfate de laminarine, dont le degré de sulfatation est supérieur ou égal à 1,9 et de préférence de 2 à 2,5 et ont le degré de polymérisation, identique à celui de la molécule naturelle, est de 20 à 30 de préférence de 23 à 25.

Le sulfate de laminarine utilisé conformément à l'invention présente, dans le cas des maladies inflammatoires relevant de réactions inflammatoires non spécifiques antigène-indépendantes, une activité anti-inflammatoire qui est comparable à celle de l'indométacine mais qui se manifeste à des concentrations moléculaires nettement plus faibles ; de plus, il n'est pas cytotoxique aux concentrations les plus élevées mises en oeuvre, et son activité anticoagulante est suffisamment faible comparée à celle de l'héparine pour ne pas constituer un inconvénient dans le cadre de l'utilisation conforme à l'invention.

Suivant un mode de réalisation avantageux de l'utilisation conforme à l'invention le sulfate de laminarine est mis en oeuvre par le biais de formes pharmaceutiques adaptées à l'administration par voie locale ou générale et plus particulièrement
1. par voie cutanée sous forme notamment d'émulsions, pommades, gels, dispositifs transdermiques
2. par voie rectale sous forme notamment de suppositoires et lavements.
3. par voie parentérale sous forme notamment de solutions injectables (sous-cutanées, intra-articulaires, intra-veineuses)
4. par voie orale sous forme notamment de comprimés, sirops, solutions ou suspensions buvables, capsules, gélules, sachets,
5. par voie pulmonaire, notamment sous forme d'aérosols et

La concentration en substance active, notamment la concentration efficace, dans le cas des susdites formes pharmaceutiques, est choisie de façon telle qu'elle rende possible l'administration quotidienne en une ou plusieurs applications ou prises, d'une quantité de substance active par kg de poids du corps du patient de 2 à 40 mg/kg/j de préférence de 10 à 25mg/kg/j dans le cas de l'administration par voie orale et de 1 à 100 mg/kg/j, de préférence de 10 à 30 mg/kg/j dans le cas d'une administration par une forme pharmaceutique à usage local.

Dans ce qui suit, on décrit d'abord un procédé de préparation du sulfate de laminarine, puis les expériences effectuées pour déterminer ses activités *in-vitro* et son activité anti-inflammatoire *in vivo.*

### I - Préparation du sulfate de laminarine

Pour préparer le sulfate de laminarine on extrait d'abord la laminarine d'une matière première constituée par des algues brunes puis on procède à la sulfatation de la laminarine ainsi extraite.

L'extraction de la laminarine peut être réalisée par mise en oeuvre du procédé décrit dans le brevet FR 92 08387.

La sulfatation de la laminarine peut être effectuée par mise en oeuvre du procédé décrit dans la publication suivante :
- Alban S, Kraus J, Franz G : Synthesis of laminarin sulfates with antocoagulant activity, Arzneim-Forsch/drug Res (1992) 42 ; 1005-1008.

Un procédé perfectionné de sulfatation de la laminarine est décrit dans la thèse de Susanne Alban, soutenue en 1993 à l'Université de Regensburg et portant le titre « Synthèse und physiologische Testung neuartiger Heparinoide ».

Un autre procédé perfectionné est décrit dans le thèse de Helmut Stibich, soutenue à l'Université de Regensburg en 2001 sous le titre « Neuartige, antiphologistisch wirksame β(1-3)-Glucansulfate: Partialsynthese und physiologische Testung ».

Ces procédés permettent d'obtenir un sulfate de laminarine hautement substitué, sans dégradation et avec une bonne reproductibilité sous de bonnes conditions du point de vue économique, tout en restant simples.

Pour aboutir à une sulfatation efficace sans dégradation des chaînes polysaccharidiques, la réaction de sulfatation doit être effectuée sous des conditions correspondant à une absence absolue d'eau.

Avant la sulfatation, la laminarine est séchée, par exemple sur pentoxide de phosphore (P₂O₅) et ensuite dissoute dans du diméthylformamide ou DMF. De par ses effets alternatifs sur le polysaccharide, le DMF a une influence activante sur la substitution. En effet, l'association du DMF polaire avec les groupes OH conduit à la coupure des liaisons hydrogène intra et inter moléculaires et à la désintégration de structures supérieures.

Pour mettre en oeuvre la réaction de sulfatation, on peut avoir recours avantageusement au complexe SO₃-pyridine.

Par suite de la coordination de l'accepteur d'électrons SO₃ avec le donneur d'électrons pyridine, la réactivité difficilement contrôlable du SO₃ qui se traduit par des réactions fortement exothermiques entraînant des dégradations, se trouve réduite. Le complexe SO₃-pyridine présente par rapport à d'autres complexes l'avantage d'être ni trop réactif ni trop stable c'est-à-dire trop lent du point de vue réaction.

En raison du fait que le degré de sulfatation obtenu est proportionnel à l'excès molaire en réactif de sulfatation et étant donné que l'on cherche à obtenir un degré de substitution supérieur à 2, on met avantageusement en oeuvre une concentration de 6 moles de SO₃-pyridine par mole de glucose.

Pour garantir l'absence d'eau, on peut travailler sous une atmosphère d'argon.

De plus, on ajoute dès le début de la réaction de la pyridine au réactif de sulfatation et ce, en quantité équimolaire en vue de capter directement l'acide sulfurique qui pourrait se former par réaction du complexe SO₃-pyridine avec l'eau. Tant la concentration de la laminarine que celle du réactif de sulfatation doivent être aussi élevées que possible, la solubilité du polysaccharide et du réactif de sulfatation, étant limitants. Pour éviter au début de la réaction un refroidissement du mélange qui pourrait entraîner des problèmes de solubilité et pour obtenir une substitution la plus régulière possible, la solution du complexe SO₃-pyridine dans le DMF pourrait ne pas être ajoutée en une seule fois mais de manière continue pendant une durée de 4 heures.

La réaction de sulfatation peut être effectuée à une température de 20 à 60°C, de préférence d'environ 40°C. Des températures plus élevées entraînent une substitution plus efficace mais, également, une dégradation des chaînes.

Après l'addition du réactif de sulfatation, on continue à agiter le mélange pendant 6 heures à 60°C. A cette température, il se produit une substitution supplémentaire sans dégradation des chaînes.

Le surnageant du mélange est séparé par décantation. Le résidu est dissous dans 2,5 M de NaOH puis mélangé avec 10 fois son volume d'éthanol à 99%. Le précipité qui se produit à une température de 4-8°C pendant la nuit est isolé puis dissous dans de la soude diluée (solution de pH d'environ 9). La solution est dialysée pour enlever les sels et les molécules de bas poids moléculaire grâce une membrane de type Spectrapor à seuil de coupure 1000 D puis amenée à un pH de 7,0 par addition de NaOH et ensuite lyophilisée. Le sulfate de laminarine résultant se présente sous forme de sel de sodium.

Le poids moléculaire de la macromolécule est déterminé en tant que volume hydrodynamique par chromatographie sur gel avec mise en oeuvre d'un système dit « Fast Protein Liquid Chromatography» ou FPLC commercialisé par la Société Pharmacia. La détection du profil d'élution (éluant : 0,1M NaCl avec 0,05% d'azide de sodium, 30ml/h) à l'aide d'un dispositif connu sous la marque Superdex 75HR10/30 (domaine de séparation 3-70kd) est effectuée par la mesure de l'indice de réfraction. Le sulfate de laminarine correspond à un pic étroit symétrique ; la largeur de ce pic est la même pour la laminarine non sulfatée et pour la laminarine sulfatée ce qui prouve que la longueur de la chaîne reste la même. Le poids moléculaire relatif est déterminé à l'aide d'une courbe standard avec des pullulans (polymère standard : 5800-853000 d, Polymer Laboratories, Separation Science Division). Par suite de la présence des groupes sulfates fortement hydratés, le volume hydrodynamique est supérieur à la masse moléculaire réelle.

Le degré de sulfatation est déterminé par voie de titration conductimétrique de l'acide libre du polysaccharide sulfaté en utilisant de la soude 0,1N ou par chromatographie ionique après hydrolyse en utilisant un système HPLC. La première méthode présente l'avantage d'être également propre à des recherches relatives à la stabilité (la consommation de soude s'accroît lorsque des groupes sulfates sont éliminés) alors que la méthode HPLC nécessite moins de substance et peut être automatisée. A titre de contrôle, il est possible de déterminer la teneur en soufre par analyse élémentaire.

Il est de plus possible de contrôler l'homogénéité de la sulfatation et la répartition des groupes sulfate sur les différentes positions dans la molécule de glucose par une forme modifiée de l'analyse de méthylation suivie d'un examen GC-MS (à savoir Chromatographie Gaz, Spectrométrie de Masse). La sulfatation est pratiquement totale, c'est-à-dire presque tous les groupes hydroxyle en position 6 sont sulfatés. Lors de la substitution des groupes OH secondaires, il n'y a pas de différence significative entre la sulfatation des groupes en position 2 et celle des groupes en position 4.

Le degré de sulfatation obtenu en procédant comme indiqué ci-dessus est supérieur à 1, 9 et plus précisément de 2 à 2,5.

Le dégré de polymérisation du sulfate de laminarine ainsi obtenu est de 20 à 30, plus précisément de 23 à 25.

L'activité anti-inflammatoire du sulfate de laminarine résulte de 3 tests in vitro et d'un test in vivo décrits ci-après.

### II-Etude in-vitro de l'activité anti-inflammatoire du sulfate de laminarine

Les trois test *in vitro* dont il va être question démontrent respectivement que le sulfate de laminarine :
⇒ inhibe l'activation du complément, processus important se produisant au début des phénomènes inflammatoires (Test 1)
⇒ inhibe la chimiotaxie des polynucléaires neutrophiles, cellules caractéristiques de l'infiltrat dans la réaction inflammatoire aiguë (Test 2) et
⇒ inhibe l'adhésion des cellules induite par les sélectines L et P (Test 3)

Dans un test supplémentaire (Test 4) on a comparé les activités anticoagulantes du sulfate de laminarine et de l'héparine.

Les trois susdits tests montrent que le sulfate de laminarine inhibe à 3 étapes différentes la réaction inflammatoire et ce de façon beaucoup plus importante que l'héparine non fractionné par exemple celle commercialisée par SIGMA sous la désignation « héparine sodique » obtenue à partir de la muqueuse de l'intestion de porc; cette héparine constitue le polysaccharide sulfaté de référence.

De plus, comparativement à celle de cette héparine, l'activité anti-coagulante du sulfate de laminarine est nettement plus faible (Test 4). Le sulfate de laminarine présente donc un rapport bénéfice / risque nettement supérieur à celui de l'héparine.

### Test 1 : Mesure de l'activité anti-complémentaire :

Le système complément joue un rôle clef dans les procédés inflammatoires induits par les infections. Les substances inhibant in vitro l'activation du complément sont des produits potentiellement anti-inflammatoires in vivo.

L'activation du complément peut se faire par la voie classique,qui fait intervenir le système antigène - anticorps, ou par la voie dite alterne. On a étudié, in vitro, l'activité anti-complémentaire du sulfate de laminarine comparativement à celle de la susdite héparine de bas poids moléculaire (HBPM) de référence.

L'évaluation de l'activité anti-complémentaire est effectuée par la mesure photométrique ou colorimétrique de l'hémoglobine libérée par la lyse des érythrocytes. On utilise des érythrocytes de mouton anticorps-sensibilisés pour la voie classique et des érythrocytes de lapin pour la voie alterne.

Dans un premier temps, le produit à tester et le système complément (issu de sérum humain) sont mis en contact l'un avec l'autre et éventuellement incubés pendant 30 minutes puis dans un second temps, ils sont mis au contact des érythrocytes pendant 45 minutes.

Plus le produit testé inhibe le complément, plus la lyse des érythrocytes est faible et moins la quantité d'hémoglobine libérée est importante.

L'activité anti-complémentaire est exprimée en IC(50) (IC signifiant «.inhibiting concentration ») qui désigne la concentration exprimée en µg de substance de test par ml de mélange de la substance de test et du sérum (µg/ml), concentration qui est nécessaire pour inhiber de 50% l'activité du complément.

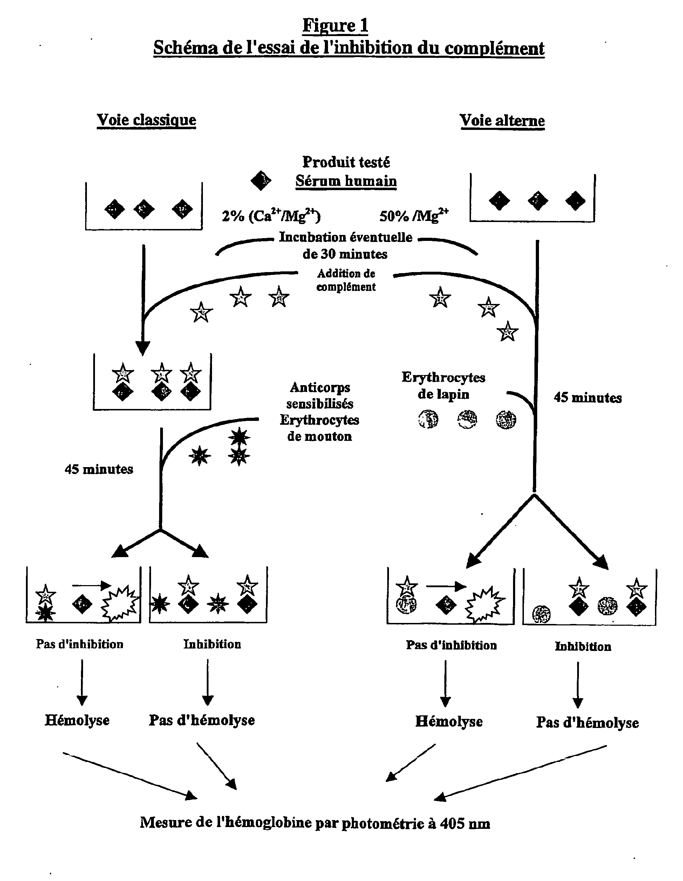

La figure 1 montre un schéma réactionnel illustrant les différentes étapes du test venant d'être décrit.

Les résultats obtenus montrent que
- dans l'inhibition de l'activation du complément par la voie classique, la valeur de l'IC (50) pour le sulfate de laminarine est de 0,54±0,06 µg/ml, celle de l'héparine étant de 43,7±1,9 ; il en résulte que par la voie classique, le sulfate de laminarine est 80 fois plus actif sur l'inhibition du complément que l'héparine, ,
- dans l'inhibition par la voie alterne, la valeur de IC (50) pour le sulfate de laminarine (sans incubation) est de 43µg/ml et celle de l'héparine de 464µg/ml ; il en résulte que par la voie alterne le sulfate de laminarine est 10 fois plus actif sur l'inhibition du complément que l'héparine,
- le sulfate de laminarine empêche la consommation du complément ; en effet, on sait que généralement dès que le sérum est décongelé, l'activation du complément débute ; cependant, les protéines du complément ne sont stables que pendant un temps limité du fait de l'inhibition par inhibiteurs endogènes,ce par suite de quoi, il se produit généralement une perte de l'activité complémentaire au cours du temps ; or on a observé dans le cas du sulfate de laminarine une prolongation de l'activité du complément dans l'activation par voie alterne de plus de 100 % quand on introduit ledit sulfate de laminarine à des concentrations faibles, (cette prolongation étant de 50% pour une dose de 2,4 µg/ml ) ce qui permet de conclure que le sulfate de laminarine empêche la consommation du complément.

Par conséquent, le sulfate de laminarine non seulement bloque l'activation du complément induite dans le test mais inhibe également son activation spontanée.

### Test 2 : Etude de l'influence sur la chimiotaxie des granulocytes

Les polymorphnucléaires neutrophiles ou PMN sont les cellules caractéristiques de l'infiltrat de l'inflammation aiguë. Le mouvement ou migration des neutrophiles de la lumière des vaisseaux vers les tissus est provoqué par des molécules dites chimiotaxines. Deux chimiotaxines sont particulièrement importantes dans le cas des polynucléaires neutrophiles, à savoir le fragment C5a du complément et l'interleukine 8 (IL-8). Le mouvement des neutrophiles peut être aléatoire (il s'agit alors de ce qu'on appelle chimiokinésie) ou orienté le long d'un gradient de chimiotaxines.

On sait que les molécules susceptibles d'inhiber cette migration des neutrophiles peuvent être utilisées dans la prévention de l'induction de la réponse inflammatoire.

On a donc étudié le pouvoir du sulfate de laminarine d'inhibiber la chimiotaxie du sulfate de laminarine ; cette étude a été faite in vitro par le test de la chambre de Boyden (on a utilisé une variante à 96 puits de la chambre de Boyden), le fragment C5a sous la forme de sérum humain activé au Zymosan étant utilisé comme chimiotaxine.

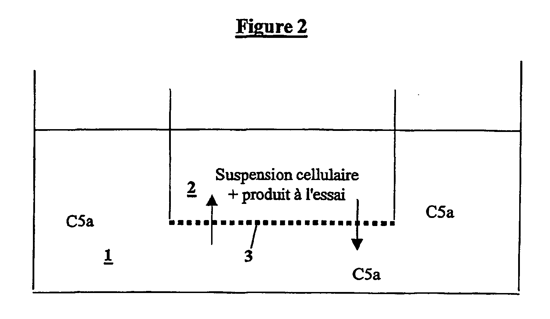

Montrée à la figure 2, la chambre de Boyden comprend 2 compartiments respectivement 1 et 2 et qui sont séparés par une membrane 3 en polycarbonate (dimension des pores : 3µm).

Les pores de cette membrane permettent le passage actif des cellules mais pas leur diffusion passive.

Les polymorphnucléaires neutrophiles PMN sont placés dans le compartiment supérieur 2 avec la substance à tester à des doses variables.

Le C5a est placé dans le compartiment inférieur.

Au cours du temps, le C5a diffuse dans le compartiment supérieur et les PMN migrent activement à travers la membrane dans le compartiment inférieur.

La mesure de l'inhibition de la chimiotaxie se fait par comptage des cellules qui ont été attirées dans le compartiment inférieur.

Pour compter les cellules, on les lyse avec du Triton X, et on quantifie ensuite la peroxydase libérée en ajoutant du sulfonate de 2,2-azino-di-[3-éthylbenzo-thiazoline] ou ABTS commercialisé par Boehringer Mannheim et en mesurant à 405 nm l'extinction du produit coloré formé.

On a trouvé qu'alors qu'en présence de 1,67µg/ml de sulfate de laminarine, la migration cellulaire est inhibée à 50% ± 6%, elle n'est pas inhibée en présence d'héparine à cette même concentration de 1,67µg/ml, une inhibition de 10% ±5% seulement étant obtenue en présence de 4,2µg/ml d'héparine.

Le pouvoir inhibiteur, dépendant de la dose, du sulfate de laminarine à l'égard de la migration des PMN étant ainsi démontré, il apparaît que ledit sulfate de laminarine peut être utilisé pour la prévention de l'induction de la réponse inflammatoire, la migration en question étant une étape important dans le processus inflammatoire.

### Test 3: Influence sur l'adhésion des cellules induite par la sélectine.

L'adhésion des cellules inflammatoires contre la paroi des vaisseaux sanguins est une condition préalable pour que les cellules migrent dans les tissus.

Il s'agit là d'un processus en plusieurs étapes qui s'effectue sous l'influence des molécules d'adhésion.

C'est sous l'influence des sélectines, une famille de molécules d'adhésion, qu'ont lieu les premiers contacts entre les leucocytes et l'endothélium.

Ces sélectines reconnaissent les ligands oligosaccharidiques de la surface cellulaire.

L'adhésion en question est labile ce qui permet aux leucocytes de se déplacer lentement le long de l'endothélium vasculaire ; ce phénomène constitue ce qu'on appelle le "rolling" des leucocytes.

La famille des sélectines comprend trois représentants, à savoir les L- sélectines, les P-sélectines et les E-sélectines ; elles sont exprimées par différentes cellules ; ainsi les L-Sélectines sont exprimées par les leucocytes, les P-sélectines par les cellules endothéliales et par les plaquettes et les E-sélectines par les cellules endothéliales vasculaires.

Etant donné que le « rolling » induit par les sélectines, constitue une condition préalable pour une adhésion solide et en fin de compte pour une extravasation des cellules inflammatoires dans les tissus, l'obtention d'une inhibition de ce processus précoce constitue une option intéressante pour une thérapie anti-inflammatoire.

On a donc étudié l'effet du sulfate de laminarine par la quantification directe des cellules liées aux sélectines immobilisées sur microplaques.

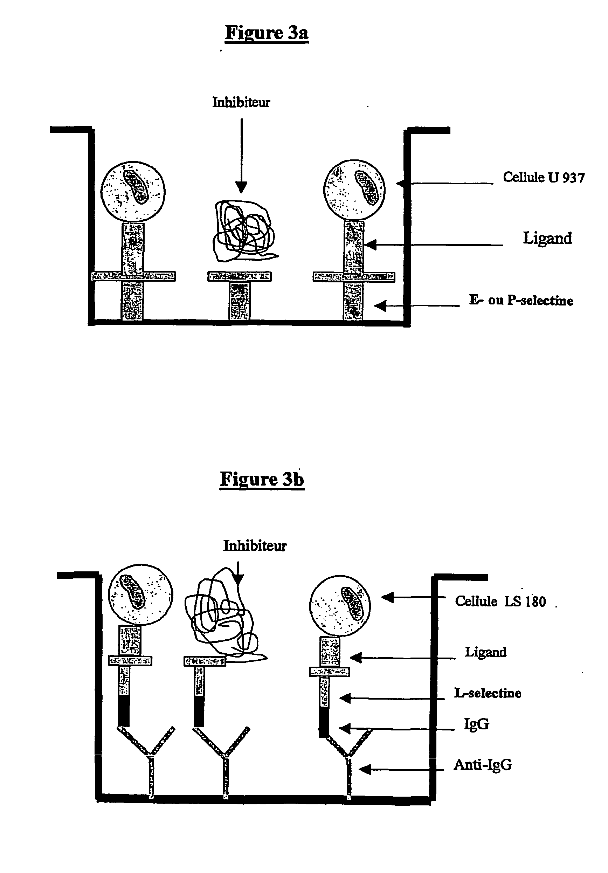

L'influence du sulfate de laminarine sur l'adhésion des cellules due aux sélectines a été étudiée tout d'abord dans des conditions statiques comme montré schématiquement aux figures 3a (E ou P-sélectines) et 3b (L-sélectines).

On dépose de la sélectine sur une plaque de microtitration ; à cet égard on utilise dans le cas de la L-sélectine la chimère humaine Fc de la L-sélectine.

Ensuite, on incube la plaque avec la substance de test et avec des cellules portant les ligands de sélectines correspondants, à savoir dans le cas de la L-sélectine la lignée cellulaire humaine LS180 de l'adénocarcinome du colon et dans le cas des sélectines P et E la lignée cellulaire humaine lymphomique U937.

On détermine le nombre de cellules liées à la plaque de microtitration après lavage et lyse subséquente de ces cellules et en déterminant leur teneur en lactate déshydrogénase grâce à une réaction enzymatique.

Les résultats obtenus sont réunis dans le tableau I.

**TABLEAU I**

| Produit testé | Concentration en µg/ml | Inhibition en % de l'adhésion cellulaire sur | |
|---|---|---|---|
| | | P-sélectine | L-sélectine |
| Sulfate de laminarine | 5 | 50±2 | 33±1 |
| | 25 | 63±3 | 89±10 |
| Héparine | 5 | 12±5 | 15±5 |
| | 25 | 30±4 | 32±3 |

### Test 4 : Etude de l'activité anti-coagulante.

Etant donné que lors de la mise à profit de l'activité inhibitrice de l'inflammation dont fait preuve le sulfate de laminarine, son activité anti-coagulante pourrait poser des problèmes du point de vue effet secondaire, on a étudié l'influence du sulfate de laminarine sur la coagulation.

L'activité anticoagulante et dépendant de la concentration du sulfate de laminarine a été déterminée en comparaison avec celle de l'héparine dans les tests de coagulations classiques APTT ou « Aktivierte partielle Thromboplastin-Zeit», de la durée de prothrombine, du test dit « HEPTEST » et de la durée de thrombine. L'APTT reflète une interaction avec le système intrinsèque de la coagulation alors que la durée de prothrombine reflète une interaction avec la coagulation extrinsèque ; le test dit « HEPTEST » est le test classique pour la mesure de l'activité inhibitrice de l'héparine à l'égard du facteur Xa et la durée de thrombine correspond à la dernière étape de la coagulation à savoir la formation de fibrines induites par la thrombine. Au contraire de celle de l'héparine, l'activité du sulfate de laminarine dans le test dit « HEPTEST » est plus de 20 fois plus faible. De même, en rapport avec la durée de prothrombine le sulfate de laminarine ne fait preuve d'aucun effet prononcé anticoagulant, comme dans le cas de l'héparine. L'activité spécifique (IU/mg) dans l'APTT représente 30% de l'activité de l'héparine et dans le cas de la durée de thrombine 60%. Pour empêcher totalement la coagulation, il convient d'appliquer dans le cas de l'APTT une concentration 4 fois plus grande et dans le cas de la durée de thrombine une concentration 20 fois plus élevée.

Dans les tests spécifiques anti-facteur Xa et anti-thrombine en utilisant des substrats chromogènes on constate que le sulfate de laminarine au- contraire de l'héparine ne présente ni une activité significative anti-facteur Xa dépendant de l'anti-thrombine ni une activité anti-thrombine. L'effet dans le cas de la durée de thrombine peut être considéré comme étant dû à une inhibition de thrombine dépendant du cofacteur II héparine. En raison d'une part de l'activité spécifique plus faible, d'autre part du profil dépendant de la concentration et d'autre part encore d'autres recherches relatives au mécanisme d'action, il est possible de considérer que dans le cas du sulfate de laminarine, le risque de saignement est sensiblement plus faible que dans le cas de l'héparine.

Pour cette raison et au vu de l'effet meilleur dans les systèmes de tests anti-inflammatoires, le profil « utilité/risque » est déplacé dans le cas du sulfate de laminarine de façon univoque dans le sens d'un effet antiinflammatoire.

Il s'ensuit que les propriétés anti-inflammatoires du sulfate de laminarine pourront être avantageusement mises à profit sans crainte d'effets secondaires indésirables sur la coagulation.

### III -Etude in vivo de l'activité anti-inflammatoire

### du sulfate de laminarine

L'activité topique, inhibitrice de l'inflammation, présentée par le sulfate de laminarine a été testée dans le modèle constitué par l'oedème de l'oreille de la souris induit avec de l'huile de croton.

En tant que témoin, on a recours à l'indométacine.

Les animaux utilisés sont des souris mâles du type Swiss CD-1 pesant de 20 à 25g.

On travaille sur trois groupes de tests effectués sur trois groupes d'animaux

Le premier groupe est un groupe témoin négatif dans lequel on applique uniquement l'agent pro-inflammatoire constitué par l'huile de croton.

Le deuxième groupe est un groupe témoin positif et comprend l'application d'huile de croton et d'indométacine (200 nMol/oreille ce qui correspond à 71,56 µg/oreille).

Le troisième groupe est relatif à l'utilisation du sulfate de laminarine et comprend l'application de 70 µg d'huile croton et de quantités croissantes de sulfate de laminarine (100, 250, 500, 750 et 1000 µg/oreille).

Dans le cas des groupes témoins, du groupe de référence et du groupe comportant la mise en oeuvre des différentes concentrations de sulfate de laminarine, on utilise chaque fois 10 souris.

Douze heures avant l'expérimentation, les animaux ne reçoivent plus de nourriture mais de l'eau à volonté.

Sur chaque oreille (surface d'environ 1 cm²) on applique 70 µg d'huile de croton en même temps que de l'indométacine ou du sulfate de laminarine ou de la dexaméthasone. Au bout de quatre heures, on découpe des échantillons d'un diamètre de 7mm et on les pèse.

Pour l'exploitation statistique on effectue une analyse de variance (ANOVA) et le test de Scheffe.

On a évalué l'intensité de l'inhibition de l'inflammation exprimée en pourcentage pour différentes quantités des produits testés, à savoir l'indométacine, la dexaméthasone et le sulfate de laminarine.

On constate qu'une concentration de 250µg de sulfate de laminarine par oreille est équipotente à celle de 72µg d'indométacine par oreille.

D'un point de vue molaire, le sulfate de laminarine est pour cette concentration, à savoir 25n Mol, neuf fois plus actif que l'indométacine à 200n Mol.

On a par ailleurs pu constater, à l'issue d'une autre expérience, qu'à la concentration équimolaire de 0,12 µmol/oreille, le sulfate de laminarine est plus efficace que la dexaméthasone.

Dans une expérience témoin, dans laquelle le sulfate de laminarine n'est pas appliqué à l'oreille simultanément à l'huile de croton, mais après cette dernière, on ne constate aucune différence du point de vue de l'activité inhibitrice.

Il s'ensuit qu'un effet faussement positif en raison d'une interaction physique avec l'huile de croton peut être considéré comme exclu.

En résumé, on a donc préparé du sulfate de laminarine ayant un degré de sulfatation supérieur ou égal à 1,9, de préférence de 2 à 2,5 et un degré de polymérisation égal à celui de la molécule naturelle, c'est-à-dire de 20 à 30 et plus préférentiellement de 23 à 25, en ayant recours à une synthèse partielle par sulfatation de laminarine extraite d'algues brunes, matière première naturelle végétale dont la source est particulièrement abondante et renouvelable.

Ce nouveau produit est en mesure d'agir de façon inhibitrice à différentes étapes de l'inflammation.

Il inhibe l'activité complémentaire aussi bien dans le cas d'une activation par la voie classique que dans le cas d'une activation alterne et interagit par conséquent, déjà à un moment très précoce, avec le processus inflammatoire.

De plus, il inhibe la chimiotaxie des granulocytes à noyaux polymorphes induite par du sérum activé au zymosan et est donc capable d'entraver la migration de cellules inflammatoires dans le tissu.

Enfin, le sulfate de laminarine empêche la liaison des cellules à la P-sélectine et à la L-sélectine et, par voie de conséquence, l'étape initiale de la cascade des adhésions.

Les effets démontrés *in vitro* relativement à une activité inhibitrice de l'inflammation sont confirmés par l'efficacité marquée dudit sulfate de laminarine dans le modèle à l'oedème de l'oreille de souris induit par l'huile de croton.

Dans ces expériences, le sulfate de laminarine est plus efficace que l'héparine.

Son activité anti-coagulante est toutefois nettement plus faible que celle de l'héparine, ce par suite de quoi le sulfate de laminarine présente un rapport « bénéfice/risque » nettement meilleur.

De plus le sulfate de laminarine ne présente aucune propriété cytotoxique ; même à la concentration la plus élevée testée à savoir 100µg/ml, on n'a constaté aucun effet cytotoxique.

Pour tester la cytotoxicité, on a utilisé les lignées cellulaires suivantes :
- MDA -MB-231 (carcinome mammaire humain)
- MCF (carcinome mammaire humain)
- U937-(lymphome humain)
- LS180 (adénocarcinome humain)

Le test mis en oeuvre est un test de prolifération de 5 jours et la détermination du nombre de cellules vivantes a été effectuée par coloration au violet cristallin.

On a également effectué un test de prolifération de 5 jours avec détermination du nombre de cellules vivantes à l'aide du test MTT.

Enfin, on a déterminé la cytotoxicité aiguë en ayant recours à un kit commercialisé par la Société Boehringer Mannheim et intitulé « Cytotoxcity détection Kit » (LDH).

A titre d'exemples non limitatifs mais correspondant à des modes de réalisation avantageux, on indique ci-après les compositions de quelques formulations pharmaceutiques à savoir celles d'une crème, d'un sachet et d'une solution pour aérosol.
1. Composition d'une crème conforme à l'invention

| | |
|---|---|
| Eau déminéralisée | 69,5 % |
| Glycérine | 5,0 % |
| Acrylate | 0,2 % |
| Sulfate de laminarine | 1,0 % |
| PEG 100 stéarate | 4,0 % |
| Alcool cétéarylique | 2,0 % |
| Conservateur | 1,0 % |
| PEG 40 stéarate | 3,0 % |
| Acétate de vitamine E | 0,5 % |
| C 12-15 alkyl benzoate | 6,5 % |
| Caprylic/capric triglycérides | 5,5 % |
| Soude | 1,8 % |

2. Composition d'un sachet pour application orale conforme à l'invention

| | |
|---|---|
| Sulfate de laminarine | 0,150 g |
| Saccharose | 2,850 g |
| Arôme orange | qs |

3. Composition d'une solution pour aérosol conforme à l'invention

| | |
|---|---|
| Sulfate de laminarine | 1 g |
| Chlorure de sodium | 0,9 g |
| Eau (pour préparation injectable) | 100 ml |

La posologie, notamment en rapport avec les susdites formes pharmaceutiques est avantageusement comme suit :
Dans le cas de la crème, on prévoit 2 à 3 applications par jour.
Dans le cas du sachet, on administre 1 à 3 sachets par jour pour un adulte.
Dans le cas de l'aérosol, on administre par jour une quantité d'aérosol correspondant à une quantité de substance active de 1500 à 7000µg.

## Revendications

1. Utilisation d'un sulfate de laminarine de degré de sulfatation supérieur ou égal à 1,9, de préférence de 2 à 2,5 et de degré de polymérisation de 20 à 30 et plus préférentiellement de 23 à 25, pour la préparation d'un médicament pour le traitement des maladies inflammatoires relevant de réactions inflammatoires non spécifiques antigène-indépendantes.

2. Utilisation selon la revendication 1 d'un sulfate de laminarine de degré de sulfatation supérieur ou égal à 1,9, de préférence de 2 à 2,5 et de degré de polymérisation de 20 à 30 et plus préférentiellement de 23 à 25, pour la préparation d'un médicament pour le traitement des maladies inflammatoires relevant de réactions inflammatoires non spécifiques antigène-indépendantes, ce médicament inhibant l'activation du complément par la voie classique ou par la voie alterne.

3. Utilisation selon la revendication 1 d'un sulfate de laminarine de degré de sulfatation supérieur ou égal à 1,9, de préférence de 2 à 2,5 et de degré de polymérisation de 20 à 30 et plus préférentiellement de 23 à 25, pour la préparation d'un médicament pour le traitement des maladies inflammatoires relevant de réactions inflammatoires non spécifiques antigène-indépendantes, ce médicament inhibant la chimiotaxie des granulocytes.

4. Utilisation selon la revendication 1 d'un sulfate de laminarine de degré de sulfatation supérieur ou égal a 1,9, de préférence de 2 à 2,5 et de degré de polymérisation de 20 à 30 et plus préférentiellement de 23 à 25, pour la préparation d'un médicament pour le traitement des maladies inflammatoires relevant de réactions inflammatoires non spécifiques antigène-indépendantes, ce médicament inhibant l'adhésion des sélectines L et P.

5. Utilisation selon l'une des revendications 1 à 4, le médicament obtenu étant adapté soit à l'administration par voie locale ou générale et plus particulièrement par voie orale sous forme notamment de comprimés, sirops, solutions ou suspensions buvables, capsules, gélules et sachets, soit à l'administration par voie parentérale sous forme notamment de solutions injectables sous-cutanées, intra-articulaires et intra-veineuse soit à l'administration par voie cutanée sous forme notamment d'émulsions, pommades, gels, dispositifs transdermiques soit encore à l'administration par voie pulmonaire sous forme notamment d'aérosols, soit enfin à l'administration par voie rectale sous forme notamment de suppositoires et de lavements.

6. Médicament pour le traitement des maladies inflammatoires relevant de réactions inflammatoires non spécifiques, c'est-à-dire antigène-indépendantes, **caracterisé par le fait qu'**il comporte en tant que substance active une concentration efficace d'un polysaccharide sulfate particulier, à savoir le sulfate de laminarine, dont le degré de sulfatation est supérieur ou égal a 1,9 et de préférence de 2 à 2,5 et dont le degré de polymérisation, identique à celui de la molécule naturelle, est de 20 à 30 de préférence de 23 à 25.

## Claims

1. Use for the preparation of a medicine for the treatment of inflammatory diseases corresponding to antigen-independent non specific inflammatory reactions of a laminarin sulphate whose degree of sulphatation is higher than or equal to 1.9, preferably from 2 to 2.5 and whose degree of polymerization is from 20 to 30, and preferably from 23 to 25.

2. Use for the preparation of a medicine for the treatment of inflammatory diseases corresponding to antigen-independent non specific inflammatory reactions and inhibiting the activation of the complement by the classical route or by the altern route, of a laminarin sulphate whose degree of sulphatation is higher than or equal to 1.9, preferably from 2 to 2.5 and whose degree of polymerization is from 20 to 30, and more preferably from 23 to 25.

3. Use for the preparation of a medicine for the treatment of inflammatory diseases corresponding to antigen-independent non specific inflammatory reactions and inhibiting the chemiotaxy of the granulocytes, of a laminarin sulphate whose degree of sulphatation is higher than or equal to 1.9, preferably from 2 to 2.5 and whose degree of polymerization is from 20 to 30, and more preferably from 23 to 25.

4. Use for the preparation of a medicine for the treatment of inflammatory diseases corresponding to antigen-independent non specific inflammatory reactions and inhibiting the adhesion of the selectins L and P, of a than or equal to 1.9, preferably from 2 to 2.5 and whose degree of polymerization is from 20 to 30, and more preferably from 23 to 25.

5. Use according to one of claims 1 to 4, the thus obtained medicine being adapted either to administration by the local or the general route and more especially by the oral route, under the form of, especially tablets, syrups, drinkable solutions or suspensions, capsules, hard capsules and sachets, or to the administration by the parenteral route under the form of, especially, subcutaneously, intra-articularly and intra-veinously injectable solutions, and to the administration by the cutaneous route under the form of, especially, emulsions, ointments, gels, transdermic devices or still to the administration by pulmonary way under the form of, especially, aerosols or finally to administration by the rectal route under the form, especially, of suppositories and rectal injections.

6. Medicine for the treatment of inflammatory diseases corresponding to non specific inflammatory reactions, that is to say antigen-independent, **characterized by** the fact that it comprises, as active substance, an efficient concentration of one particular sulphated polysaccharide, i.e. laminarin sulphate, whose degree of sulphatation is higher than or equal to 1.9 and preferably from 2 to 2.5 and whose degree of polymerization, which is identical to that of the natural molecule, is from 20 to 30, and more preferably from 23 to 25.

## Patentansprüche

1. Verwendung von Laminarinsulfat mit einem Sulfatisierungsgrad höher als oder gleich 1,9, vorzugsweise 2 bis 2,5, und mit einem Polymerisationsgrad von 20 bis 30 und bevorzugter von 23 bis 25 zur Herstellung eines Medikaments für die Behandlung von entzündlichen Krankheiten, die sich aus unspezifischen, antigenunabhängigen entzündlichen Reaktionen ergeben.

2. Verwendung nach Anspruch 1 von Laminarinsulfat mit einem Sulfatisierungsgrad höher als oder gleich 1,9, vorzugsweise 2 bis 2,5, und einem Polymerisationsgrad von 20 bis 30 und bevorzugter von 23 bis 25 für die Herstellung eines Medikaments zur Behandlung von entzündlichen Krankheiten, die sich aus unspezifischen, antigenunabhängigen entzündlichen Reaktionen ergeben, wobei dieses Medikament die Aktivierung des Komplements über den klassischen Weg oder über den alternativen Weg hemmt.

3. Verwendung nach Anspruch 1 von Laminarinsulfat mit einem Sulfatisierungsgrad höher als oder gleich 1,9, vorzugsweise 2 bis 2,5, und einem Polymerisationsgrad von 20 bis 30 und bevorzugter von 23 bis 25 für die Herstellung eines Medikaments zur Behandlung von entzündlichen Krankheiten, die sich aus unspezifischen, antigenunabhängigen entzündlichen Reaktionen ergeben, wobei dieses Medikament die Chemotaxie der Granulozyten hemmt.

4. Verwendung nach Anspruch 1 von Laminarinsulfat mit einem Sulfatisierungsgrad höher als oder gleich 1,9, vorzugsweise 2 bis 2,5, und einem Polymerisationsgrad von 20 bis 30 und bevorzugter 23 bis 25 für die Herstellung eines Medikaments zur Behandlung von entzündlichen Krankheiten, die sich aus unspezifischen, antigenunabhängigen entzündlichen Reaktionen ergeben, wobei dieses Medikament die Adhäsion der Selektine L und P hemmt.

5. Verwendung nach einem der Ansprüche 1 bis 4, wobei das erhaltene Medikament entweder für die Verabreichung auf lokalem oder allgemeinem Weg und insbesondere auf oralem Weg insbesondere in Form von Tabletten, Sirups, Lösungen oder trinkbaren Suspensionen, Kapseln, Gelatinekapseln und Beuteln, oder für die Verabreichung auf parenteralem Weg insbesondere in Form von injizierbaren subkutanen, intraartikularen und intravenösen Lösungen oder für die Verabreichung auf kutanem Weg insbesondere in Form von Emulsionen, Pomaden, Gels, transdermalen Vorrichtungen oder für die Verabreichung auf pulmonarem Weg insbesondere in Form von Aerosolen oder für die Verabreichung auf rektalem Weg insbesondere in Form von Suppositorien und Einläufen ausgelegt ist.

6. Medikament für die Behandlung von entzündlichen Krankheiten, die sich aus unspezifischen, d.h. antigenunabhängigen entzündlichen Reaktionen ergeben, **dadurch gekennzeichnet, dass** es als Wirksubstanz eine wirksame Konzentration eines besonderen Polysaccharidsulfats, und zwar des Laminarinsulfats, enthält, dessen Sulfatisierungsgrad höher als oder gleich 1,9 ist, vorzugsweise 2 bis 2,5, und dessen Polymerisationsgrad, der mit dem des natürlichen Moleküls identisch ist, 20 bis 30 und vorzugsweise 23 bis 25 beträgt.
